# EUROPÄISCHE PATENTSCHRIFT

(11) **EP 1 458 440 B1**
(45) Veröffentlichungstag und Bekanntmachung des Hinweises auf die Patenterteilung: **11.04.2007**
(21) Anmeldenummer: 02781047.2
(22) Anmeldetag: 11.12.2002
(51) Int. Cl.: A61M 5/315, A61M 5/24

(54) **Injektionsgerät mit endpositionsblockiertem Dosiseinstellglied**
Injection device comprising a dose-metering member, whose end position is blocked
Appareil d'injection comprenant un element de reglage de dose à position finale bloqueé

(30) Priorität: 21.12.2001 DE 10163328; 11.06.2002 DE 20209051 U
(43) Veröffentlichungstag der Anmeldung: 22.09.2004
(73) Patentinhaber: TecPharma Licensing AG, 3401 Burgdorf (CH)
(72) Erfinder: SOMMER, Christoph, CH-3435 Ramsei (CH); KIRCHHOFER, Fritz, CH-3454 Sumiswald (CH); SCHÄR, Michael, CH-4543 Deitingen (CH)
(74) Vertreter: Schwabe - Sandmair - Marx
(86) Internationale Anmeldenummer: PCT/CH2002/000684
(87) Internationale Veröffentlichungsnummer: WO 2003/053499

(56) Entgegenhaltungen:
- EP-A- 0 879 610
- EP-A- 0 897 728
- WO-A-99/38554
- DE-A- 4 112 259
- US-A- 5 514 097
- US-A- 5 584 815

## Beschreibung

Die Erfindung betrifft bei einem Injektionsgerät für die dosierte Verabreichung eines injizierbaren Produkts die Dosierung.

In der Verabreichung von Produkten, insbesondere in medizinischen Anwendungen, kommt der genauen Dosierung des Produkts eine große Bedeutung zu. Bei Injektionsgeräten wird die Dosierung im Allgemeinen mit Hilfe eines Dosiseinstellglieds vorgenommen, das mit einer Fördereinrichtung so gekoppelt ist, dass mit Hilfe des Dosiseinstellglieds die zu verabreichende Dosis ausgewählt und bei Betätigung durch die Wirkung der Fördereinrichtung ausgeschüttet wird. Durch unsachgemäße Handhabung können aus dem Vorgang der Dosisauswahl Probleme entstehen. Auf unsachgemäße Handhabung zurückführbare Probleme treten in erhöhtem Maße in der Selbstverabreichung auf, wie sie beispielsweise in der Verabreichung von Insulin oder Wachstumshormonen üblich ist. So kann eine durch unsachgemäße Ausführung der Dosierung hervorgerufene Reaktionsbewegung eines an der Dosierung beteiligten Elements zu einer Fehldosierung führen, die von dem Verwender nicht bemerkt wird.

Aus der EP 0 897 728 A1 ist beispielsweise ein Injektionsgerät bekannt, dessen Fördereinrichtung einen Kolben und einen auf den Kolben wirkende Kolbenstange mit einem Dosiergewinde umfasst. Die Kolbenstange ist mit einer Dosiermutter in einem Gewindeeingriff und mit einer zweigeteilten Dosisanzeigehülse für die Auswahl der Produktdosis in einem verdrehgesicherten Eingriff. Die Kolbenstange ist in einem Gehäuse des Injektionsgeräts um ihre Längsachse drehbar und entlang der Längsachse axial bewegbar gelagert. Die Dosiermutter wird von dem Gehäuse entlang der Drehachse axial bewegbar, aber um die Drehachse nicht verdrehbar gelagert. Durch eine Dosierdrehbewegung der Dosisanzeigehülse wird die Kolbenstange um ihre Drehachse verdreht und aufgrund des Gewindeeingriffs mit der Dosiermutter im Gehäuse in eine Vorschubrichtung axial bewegt. Die Dosisanzeigehülse umfasst zwei relativ zueinander verdrehbare Hülsenabschnitte, nämlich einen Hülsenabschnitt, der die auswählbaren Einzel-Dosiseinheiten anzeigt und einen weiteren Hülsenabschnitt, der die auswählbaren Dosiseinheiten in einer 10er-Teilung anzeigt. Die beiden Hülsenabschnitte sind in diskreten Drehstellungen mit Hilfe eines Splints verdrehgesichert miteinander verbunden. Der Hülsenabschnitt mit der 10er-Teilung ist zwischen zwei Endpositionen verdrehbar, wobei durch den Splint in beiden Endpositionen eine verdrehgesicherte Verbindung mit dem die Einzeldosen anzeigenden Hülsenabschnitt besteht. Der verdrehgesicherte Eingriff zwischen der Kolbenstange und der Dosisanzeigehülse ist gelöst, wenn die Kolbenstange nach Ausführung eines Ausschütthubs eine vordere, axiale Endposition einnimmt, und wird bei der Rückführung der Kolbenstange in eine hintere, axiale Endposition wieder hergestellt. Zu einer unbemerkten Fehldosierung kann es beispielsweise dann kommen, wenn die Dosisanzeigehülse sich in einer ihrer Endpositionen befindet, wenn der verdrehgesicherte Eingriff mit der Kolbenstange hergestellt wird. Für das Herstellen der verdrehgesicherten Verbindung müssen sich nämlich die hierfür erforderlichen Eingriffselemente der Kolbenstange und der Dosisanzeigehülse "fangen", d.h. sie richten sich für den Eingriff aneinander aus.

Die EP 0 879 610 A2 offenbart ein Injektionsgerät mit einem drehbaren Dosiseinstellglied. Eine Maximaldosis-Position des Dosiseinstellglieds wird durch einen Translationsanschlag begrenzt.

Injektionsgeräte mit Dosiseinstellgliedern, die zum Einstellen der Dosis je gegen einen Verdrehanschlag verdrehbeweglich sind, gehen aus der US 5,584,815 B und der DE 41 12 259 A1 hervor. Ein Verdrehanschlag ist allerdings nur für jeweils eine der beiden Endpositionen des jeweiligen Dosiseinstellglieds vorgesehen.

Die WO 99/38554 A1 beschreibt ein gattungsgemäßes Injektionsgerät.

Es ist eine Aufgabe der Erfindung, die Gefahr von unbemerkten Fehldosierungen bei Injektionsgeräten zu verringern.

Die Erfindung hat ein Injektionsgerät zum Gegenstand, das ein Gehäuse, eine Fördereinrichtung, ein mit der Fördereinrichtung gekoppeltes Dosiseinsteilglied und eine Verdrehsicherungseinrichtung umfasst. Das Gehäuse kann selbst unmittelbar ein Reservoir für ein injizierbares Produkt bilden. Vorzugsweise bildet das Gehäuse jedoch eine Aufnahme für ein das Reservoir bildendes Behältnis, vorzugsweise eine Ampulle. Mit Hilfe des Dosiseinstellglieds wird eine zu verabreichende Produktdosis ausgewählt. Die Fördereinrichtung dient der Ausschüttung der ausgewählten Produktdosis, indem sie auf das in dem Reservoir befindliche Produkt in geeigneter Weise einwirkt, wofür sie vorzugsweise eine Verdrängerpumpe bildet. Das Dosiseinstellglied ist für die Auswahl der Produktdosis relativ zu dem Gehäuse rotatorisch in eine erste Drehrichtung und in eine der ersten Drehrichtung entgegengesetzte zweite Drehrichtung bewegbar. Vorzugsweise wird es unmittelbar von dem Gehäuse bewegbar gelagert. Diese rotatorische Bewegung, die im Folgenden auch als Dosierdrehbewegung bezeichnet wird, ist in die erste Drehrichtung bis in eine Endposition ausführbar. Die Fördereinrichtung und das Dosiseinstellglied sind so miteinander gekoppelt, vorzugsweise mechanisch, dass die Fördereinrichtung die mittels des Dosiseinstellglieds ausgewählte Produktdosis durch einen Auslass des Reservoirs fördert und in diesem Sinne ausschüttet, wenn die Fördereinrichtung betätigt wird. Die Fördereinrichtung kann grundsätzlich zwar angetrieben sein, beispielsweise motorisch oder mittels Federkraft, und lediglich manuell ausgelöst werden, bevorzugt weist die Fördereinrichtung jedoch keinen eigenen Antrieb auf, sondern es wird die für die Förderung erforderliche Bewegung der Fördereinrichtung manuell bewirkt.

Die Verdrehsicherungseinrichtung umfasst einen Verdrehanschlag und einen Verdrehgegenanschlag, von denen der eine von dem Gehäuse und der andere von dem Dosiseinstellglied gebildet wird. Der Verdrehanschlag und der Verdrehgegenanschlag sind in der genannten Endposition gegeneinander in die erste Drehrichtung auf Anschlag, wodurch eine rotatorische Bewegung des Dosiseinstellglieds in die erste Drehrichtung über die Endposition hinaus verhindert wird. Die von den Anschlägen gebildete Blockierung wirkt formschlüssig und kann ohne Zerstörung der die Anschläge bildenden Teile nicht überwunden werden.

Das Dosiseinstellglied steht mit der Fördereinrichtung vorteilhafterweise permanent in einem mechanischen Eingriff, der während des bestimmungsgemäßen Gebrauchs des Injektionsgeräts nicht gelöst wird.

In bevorzugter Ausführung ist das Dosiseinstellglied in einem Stück oder aus in Bezug auf seine Dosierdrehbewegung miteinander verdrehgesichert verbundenen Stücken gebildet.

Die Fördereinrichtung umfasst in bevorzugter Ausführung eine Abtriebseinrichtung, die eine Ausschüttbewegung in eine Vorschubrichtung entlang einer Translationsachse ausführt, um die ausgewählte Produktdosis auszuschütten, und eine Antriebseinrichtung, die die Ausführung der Ausschüttbewegung bewirkt. Die Abtriebseinrichtung und die Antriebseinrichtung können in einem Stück gebildet sein, bevorzugter werden sie jedoch von separaten, miteinander mechanisch in Eingriff befindlichen Teilen der Fördereinrichtung gebildet. Das Dosiseinstellglied ist mit der Abtriebseinrichtung so gekoppelt, dass eine Dosierdrehbewegung, die das Dosiseinstellglied und die Abtriebseinrichtung um die Translationsachse relativ zueinander ausführen, eine translatorische Dosierbewegung des Dosiseinstellglieds entlang der Translationsachse relativ zu der Abtriebseinrichtung und dem Gehäuse bewirkt. Ferner ist der Eingriff bevorzugt so gestaltet, dass das Dosiseinstellglied von der Abtriebseinrichtung bei der Ausschüttbewegung mitgenommen wird.

Die Abtriebseinrichtung kann insbesondere eine Kolbenstange sein, die mit einem Dosiergewinde versehen ist. In dieser Ausführung gehört im zusammengebauten Zustand des Injektionsgeräts auch ein Kolben zur Fördereinrichtung, der in dem Reservoir angeordnet ist, das Reservoir zur Kolbenstange hin fluiddicht verschließt und bei der Ausschüttbewegung der Kolbenstange entlang der Translationsachse auf einen Auslass des Reservoirs zu verschoben wird, so dass die ausgewählte Produktdosis durch den Reservoirauslass ausgeschüttet wird. Das Dosiseinstellglied weist vorzugsweise ein Dosiergegengewinde auf, das mit dem Dosiergewinde der Kolbenstange in einem Gewindeeingriff ist, der die Kopplung zwischen der Fördereinrichtung und dem Dosiseinstellglied bildet.

Die eine der von der Verdrehsicherungseinrichtung blockierte Endposition des Dosiseinstellglieds ist eine Position die das Dosiseinstellglied bei der Auswahl einer Minimaldosis einnimmt, wobei die Minimaldosis die Nulldosis oder auch grundsätzlich eine hiervon sich unterscheidende Minimaldosis sein kann. Die andere Endposition ist eine Position, die das Dosiseinstellglied einnimmt, wenn die maximal auswählbare Dosis, d.h. die Maximaldosis, ausgewählt wird. Das Dosiseinstellglied ist sowohl gegen ein Überdrehen über die der Minimaldosis entsprechende Endposition als auch gegen ein Überdrehen über die der Maximaldosis entsprechende Endposition durch die Verdrehsicherungseinrichtung blockiert. Soweit im Folgenden zwischen den beiden Endpositionen unterschieden werden soll, wird die der auswählbaren Minimaldosis entsprechende Endposition als "Stop Minimaldosis" und die der auswählbaren Maximaldosis entsprechende Endposition als "Stop Maximaldosis" bezeichnet.

Die Verdrehsicherungseinrichtung bildet für beide Endpositionen je wenigstens ein Anschlagpaar aus einem Verdrehanschlag und einem Verdrehgegenanschlag. Der wenigstens eine Verdrehanschlag und wenigstens eine Verdrehgegenanschlag für die eine der beiden Endpositionen können identisch ausgebildet sein wie der wenigstens eine Verdrehanschlag und wenigstens eine Verdrehgegenanschlag für die andere der beiden Endpositionen. Die Anschlagpaare können jedoch auch unterschiedlich ausgebildet werden, das beispielsweise dann von Vorteil sein kann, wenn die geometrischen Verhältnisse in der Umgebung, in der das jeweilige Anschlagpaar zusammenwirkt, sich voneinander unterscheiden oder eine unterschiedliche Ausbildung möglicherweise sogar erfordern.

Der Dosierdrehbewegung des Dosiseinstellglieds ist vorzugsweise eine translatorische Bewegung überlagert, so dass sich die Dosierbewegung des Dosiseinstellglieds aus einer rotatorischen und einer translatorischen Bewegungskomponente zusammensetzt. Falls die Fördereinrichtung eine Kolbenstange mit Dosiergewinde umfasst und das Dosiseinstellglied und die Kolbenstange miteinander in einem Gewindeeingriff sind, ist die Kolbenstange in dieser Ausführung mit dem Gehäuse vorzugsweise um ihre Längsachse verdrehgesichert und entlang der Längsachse translatorisch hin und her bewegbar verbunden, während das Dosiseinstellglied relativ zu dem Gehäuse und der Kolbenstange eine exakte Dosierbewegung aus Rotation und überlagerter Translation ausführen kann. Die Längsachse der Kolbenstange bildet gleichzeitig die genannte Translationsachse der Fördereinrichtung, die Gewindeachse des Gewindeeingriffs und die Drehachse des Dosiseinstellglieds.

Obgleich eine Verdrehsicherungseinrichtung, die das Dosiseinstellglied sowohl in der Position "Stop Minimaldosis" als auch in der Position "Stop Maximaldosis" blockiert, bereits ausreicht, um den Verstellweg des Dosiseinstellglieds zwischen diesen beiden Endpositionen zu begrenzen, sind vorzugsweise zusätzlich auch noch in beide Axialrichtungen wirkende Anschläge für das Dosiseinstellglied gebildet. In solch einer Ausführung bestünde ohne die Verdrehsicherungseinrichtung die Gefahr, dass der Verwender bei dem Vorgang der Dosisauswahl das Dosiseinstellglied mit solch einer Kraft gegen einen der axial wirkenden Anschläge drückt, sozusagen das Dosiseinstellglied gegen den entsprechenden Axialanschlag schraubt, dass die Kolbenstange als Reaktionsglied auf den Kolben zu oder von dem Kolben weg bewegt wird. Hierdurch entsteht die Gefahr, dass eine andere Dosis gewählt als durch eine Anzeige des Injektionsgeräts angezeigt wird. Durch die erfindungsgemäße Verdrehsicherungseinrichtung wird dies jedoch von vornherein verhindert.

Ist die Dosierbewegung des Dosiseinstellglieds eine aus Translation und Rotation zusammengesetzte Bewegung, so kann mit einem einstückigen oder aus verdrehgesichert miteinander verbundenen Stücken zusammengesetztes Dosiseinstellglied über seinen maximal möglichen Verstellweg vorteilhafterweise mehr als eine Umdrehung um seine Drehachse ausführen. Hierdurch kann pro Ausschüttung im Vergleich zu einem nur eine Umdrehung oder weniger umfassenden Verstellweg entweder die Dosisauswahl verfeinert oder die auswählbare Dosis pro Ausschüttung vergrößert werden.

Vorzugsweise sind um die Drehachse der Dosierdrehbewegung des Dosiseinstellglieds mehrere Verdrehanschläge und/oder mehrere Verdrehgegenanschläge bevorzugt gleichmäßig verteilt ausgebildet. Grundsätzlich würde die Ausbildung eines einzigen Verdrehanschlags und eines einzigen Verdrehgegenanschlags für die Blockierung in der Endposition oder je ein solches Anschlagpaar für jede blockierte Endposition genügen.

In einer vorteilhaften Ausführung bildet ein elastisch nachgiebiger Schnapper den Verdrehanschlag oder bildet je ein elastisch nachgiebiger Schnapper je einen von mehreren Verdrehanschlägen. Besonders vorteilhaft ist die Ausbildung des wenigstens einen Schnappers als biegeelastische Zunge. Obwohl es grundsätzlich möglich wäre, dass die Zunge in eine der Drehrichtungen des Dosiseinstellglieds oder in beide Drehrichtungen biegeelastisch ist, wird einer Zunge, die in keine der Drehrichtungen nachgiebig ist, der Vorzug gegeben. Die Zunge sollte in eine Richtung nachgiebig sein, die eine in Bezug auf die Drehachse der Dosierdrehbewegung radiale Richtungskomponente hat oder noch bevorzugter radial zu der Drehachse weist. Solch ein als Zunge gebildeter Schnapper ragt vorzugsweise tangential, d.h. in eine der Drehrichtungen des Dosiseinstellglieds, von dem Dosiseinstellglied oder dem Gehäuse ab. Er sollte des Weiteren zu der Drehachse der Dosierdrehbewegung hin gekrümmt sein, vorzugsweise mit dem konstanten Krümmungsradius seines radialen Abstands von der Drehachse. Der Schnapper bildet den wenigstens einen Verdrehanschlag als Anschlagfläche, die in die erste Drehrichtung weist, vorzugsweise an einem in Bezug auf die erste Drehrichtung vorderen, freien Schnapperende. Der Schnapper kann in axialer Richtung ebenfalls elastisch nachgiebig sein. Bevorzugt ist er in axialer Richtung jedoch nicht nachgiebig, zumindest nicht unter den im Betrieb auftretenden Axialkräften.

Vorzugsweise umgeben das Gehäuse und das Dosiseinstellglied einander zumindest in je einem axialen Abschnitt, vorzugsweise je einem Hülsenabschnitt, wobei in dem Abschnitt des Gehäuses und in dem Abschnitt des Dosiseinstellglieds der wenigstens eine Verdrehanschlag und der wenigstens eine Verdrehgegenanschlag gebildet sind. Wird der wenigstens eine Verdrehanschlag von einem elastischen Schnapper gebildet, so ist der an einem ersten der Abschnitte abgestützte Schnapper radial gegen den anderen, zweiten der Abschnitte elastisch gespannt. Der wenigstens eine Verdrehgegenanschlag ragt von dem zweiten der Abschnitte in Richtung auf den ersten der Abschnitte vor und ist vorzugsweise starr, d.h. nicht nachgiebig. Der Verdrehgegenanschlag kann auch von einer Seitenwandung einer Ausnehmung in dem zweiten der Abschnitte gebildet werden, in die der elastisch gespannte Schnapper vorschnappen kann, so dass er bei Erreichen der blockierten Endposition in die erste Drehrichtung auf Anschlag gegen die betreffende Seitenwandung der Ausnehmung zu liegen kommt.

Vorstehend wurde zwar die bevorzugte Ausführung für die Bildung des wenigstens einen Verdrehanschlags und des wenigstens einen Verdrehgegenanschlags als elastischer Schnapper einerseits und starrer Gegenanschlag andererseits hervorgehoben, grundsätzlich können jedoch sowohl der Verdrehanschlag als auch der Verdrehgegenanschlag je als elastischer Schnapper oder als starrer Anschlag gebildet sein. Durch eine elastische Nachgiebigkeit von wenigstens einem der beiden Funktionsteile kann jedoch eine Behinderung in die nicht blockierte Drehrichtung auf einfache Weise vermieden werden. Die Ausbildung als biegeelastischer Schnapper, der in die Drehrichtung starr ist, ist besonders zu bevorzugen, da hier der genannte Vorteil sich noch zu dem Vorteil der steifen und dadurch sicheren Blockierung addiert. Auch eine in Bezug auf die blockierte Drehrichtung starre Ausbildung des wenigstens einen Verdrehanschlags und des wenigstens einen Verdrehgegenanschlags ist möglich. In solch einer Ausbildung der Verdrehsicherungseinrichtung wird es bevorzugt, wenn der wenigstens eine Verdrehanschlag und der wenigstens eine Verdrehgegenanschlag in und gegen die Vorschubrichtung aufeinander zuragen.

Das Injektionsgerät kann insbesondere ein Injektionspen, vorzugsweise ein Semidisposable-Pen, sein. Bei solchen Semidisposable-Pen bilden ein als Einwegmodul konzipiertes Reservoirmodul und ein wiederholt verwendbares Dosier- und Betätigungsmodul im zusammengesetzten Zustand das Injektionsgerät. Bei dem Zusammensetzen dieser beiden Module wird das Dosiseinstellglied mit einem Dosier- und Betätigungselement des Dosier- und Betätigungsmoduls verbunden. Bei solchen Semidisposable-Pens verhindert die erfindungsgemäße Verdrehsicherungseinrichtung vorteilhafterweise, dass durch das Herstellen der Verbindung Drehmomente auf die Fördereinrichtung ausgeübt werden können, die Reaktionsbewegungen auslösen könnten.

Weitere bevorzugte Merkmale der Erfindung werden durch die Unteransprüche und die Kombinationen von mehreren Unteransprüchen offenbart, auf die diesbezüglich Verwiesen wird.

Nachfolgend werden Ausführungsbeispiele der Erfindung anhand von Figuren erläutert. Die an den Ausführungsbeispielen offenbar werdenden Merkmale bilden je einzeln und in jeder Merkmalskombination die Gegenstände der Ansprüche vorteilhaft weiter. Es zeigen:
- Figur 1: zwei Teile eines Reservoirmoduls eines Injektionsgeräts,
- Figur 2: das Injektionsgerät in einem Längsschnitt,
- Figur 3: ein Dosiseinstellglied und einen Hülsenkörper eines Gehäuses des Injektionsgeräts in perspektivischer Darstellung nach einem ersten Ausführungsbeispiel,
- Figur 4: das Dosiseinstellglied in einer anderen perspektivischen Darstellung,
- Figur 5: den Hülsenkörper und das Dosiseinstellglied in einer blockierten Endposition des Dosiseinstellglieds in einem Längsschnitt,
- Figur 6: eine perspektivische Sicht auf einen das Dosiseinstellglied in der Endposition der Figur 5 blockierenden Teil einer Verdrehsicherungseinrichtung,
- Figur 7: den Hülsenkörper und das Dosiseinstellglied unmittelbar vor dem Erreichen einer blockierten anderen Endposition des Dosiseinstellglieds in einem Längsschnitt,
- Figur 8: eine perspektivische Sicht auf einen das Dosiseinstellglied in der anderen Endposition blockierenden Teil der Verdrehsicherungseinrichtung,
- Figur 9: eine perspektivische Darstellung eines Hülsenkörpers des Gehäuses nach einem zweiten Ausführungsbeispiel,
- Figur 10: den Hülsenkörper des zweiten Ausführungsbeispiels in einer Seitenansicht,
- Figur 11: den Hülsenkörper des zweiten Ausführungsbeispiels in einer Frontansicht,
- Figur 12: eine perspektivische Darstellung eines Dosiseinstellglieds nach dem zweiten Ausführungsbeispiel,
- Figur 13: das Dosiseinstellglied des zweiten Ausführungsbeispiels in einem Längsschnitt,
- Figur 14: das Dosiseinstellglied des zweiten Ausführungsbeispiels in einer Seitenansicht und
- Figur 15: das Dosiseinstellglied des zweiten Ausführungsbeispiels in einer Frontansicht.

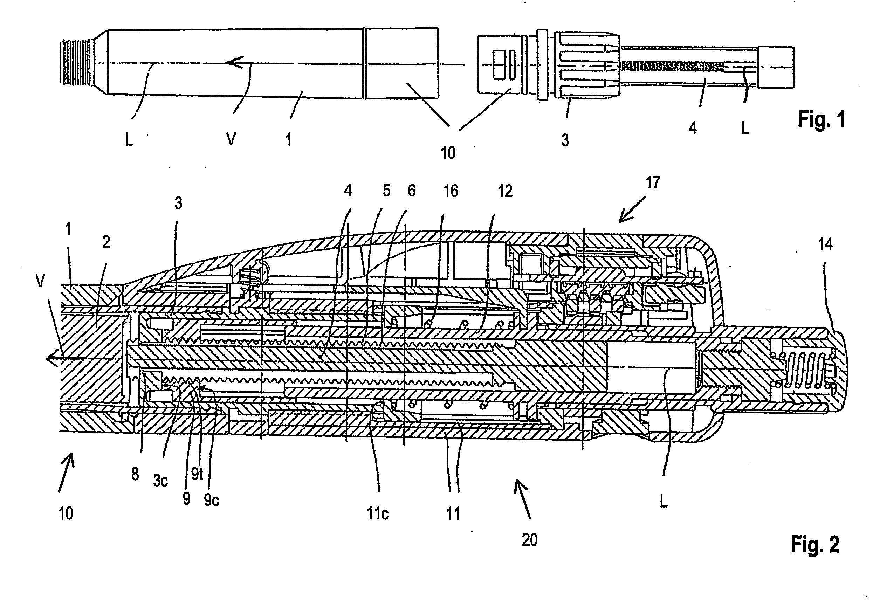

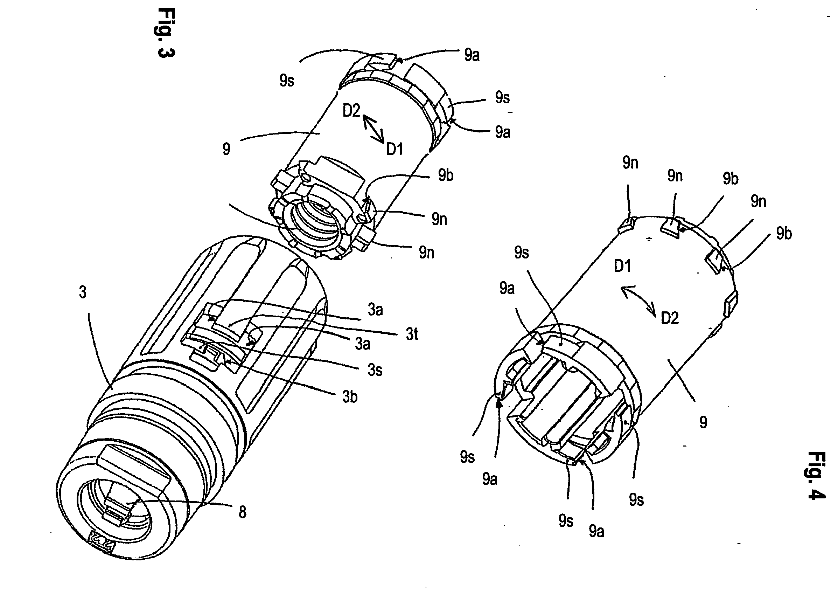

Figur 1 zeigt in einer Ansicht ein Reservoirteil 1 und einen Mechanikhalter 3, die miteinander verbunden werden, um ein Reservoirmodul 10 eines Injektionsgeräts zu bilden. Das Reservoirteil 1 ist im Wesentlichen ein im Querschnitt kreisförmiger Hohlzylinder, der an seinem vorderen Ende einen Verbindungsbereich für eine Verbindung mit einem Nadelhalter für eine Injektionsnadel aufweist. Das Reservoirteil 1 dient der Aufnahme eines Reservoirbehältnisses, das vorzugsweise von einer Ampulle gebildet wird. Das Reservoirbehältnis ist mit einem injizierbaren Produkt, beispielsweise Insulin oder ein Wachstumshormon, gefüllt. Ein Auslass an dem vorderen Ende des Reservoirbehältnisses wird von einer Membran fluiddicht verschlossen. Bei der Befestigung des Nadelhalters an dem vorderen Ende des Reservoirteils 1 durchsticht ein rückwärtiger Teil der Injektionsnadel die Membran, so dass eine Fluidverbindung zwischen der Spitze der Injektionsnadel und dem Reservoir hergestellt ist. Ein dem Auslass axial gegenüberliegendes, hinteres Ende des Reservoirbehältnisses wird fluiddicht von einem Kolben verschlossen, der entlang einer Längsachse L auf den Auslass des Reservoirbehältnisses zu verschiebbar ist, um Produkt aus dem Reservoirbehältnis zu verdrängen. In Figur 1 ist eine Kolbenstange 4 erkennbar, die an einem von dem Reservoirteil 1 abgewandten, hinteren Ende des Mechanikhalters 3 in den Mechanikhalter 3 hineinragt und von dem Mechanikhalter 3 in eine entlang der Längsachse L weisende Vorschubrichtung V auf den Reservoirauslass zu bewegbar gelagert wird.

Figur 2 zeigt einen hinteren Teil des Injektionsgeräts in einem Längsschnitt. Das Injektionsgerät wird von dem Reservoirmodul 10 und einem Dosier- und Betätigungsmodul 20 gebildet. Zu erkennen ist in Figur 1 das hintere Ende des Reservoirmoduls 10. Das Reservoirbehältnis ist vollständig mit Produkt gefüllt, so dass gerade noch der hintere Teil des Kolbens 2 zu sehen ist. Die Kolbenstange 4 bewirkt den Vorschub des Kolbens 2 in die Vorschubrichtung V auf den Reservoirauslass zu, wobei sie mit ihrem vorderen Ende gegen den Kolben 2 drückt. Die Längsachse L ist die Translationsachse des Kolbens 2 und der Kolbenstange 4. Die Kolbenstange 4 wird von dem Mechanikhalter 3 so gehalten, dass sie nach Überwindung eines gewissen Widerstands in die Vorschubrichtung V, nicht jedoch entgegen der Vorschubrichtung V bewegt werden kann. Die Rückwärtsbewegung der Kolbenstange 4 entgegen der Vorschubrichtung V wird von einer Blockiereinrichtung 8 verhindert. Zwei radial aufeinander zuragende, elastische Sperrzungen bilden die Blockiereinrichtung 8. Die Kolbenstange weist den Sperrzungen gegenüberliegend zwei axial erstreckt Reihen von Sägezähnen 6 auf, in die die Sperrzungen der Blockiereinrichtung 8 eingreifen. Die Sägezähne 6 sind in Vorschubrichtung V gepfeilt, um die Translationsbewegung der Kolbenstange 4 in die Vorschubrichtung V zuzulassen. Ihre rückwärtigen Enden sind jedoch so geformt, dass durch den Eingriff der Sperrzungen 8 die Rückwärtsbewegung verhindert wird.

In dem Mechanikhalter 3 ist ferner ein Dosiseinstellglied 9 aufgenommen. Das Dosiseinstellglied 9 ist als Gewindemutter ausgebildet und steht mit seinem Innengewinde 9t mit einem Dosiergewinde 5 der Kolbenstange 4 in einem Gewindeeingriff. Die Kolbenstange 4 wird von dem Mechanikhalter 3 in Bezug auf die Längsachse L verdrehgesichert in Vorschubrichtung V geradgeführt. Das Dosiseinstellglied 9 wird von dem Mechanikhalter 3 ebenfalls axial geführt, allerdings kann das Dosiseinstellglied 9 relativ zu dem Mechanikhalter 3 und der Kolbenstange 4 um die Längsachse L eine rotatorische Bewegung ausführen. Die Kolbenstange 4 und das Dosiseinstellglied 9 bilden einen Spindeltrieb, um die zu verabreichende Produktdosis auszuwählen.

Das Reservoirteil 1 und der Mechanikhalter 3 sind verdreh- und verschiebegesichert miteinander verbunden und bilden zusammen das Reservoirmodul 10 des Injektionsgeräts. Das Reservoirmodul 10 umfasst daher auch die mittels der Blockiereinrichtung 8 gehaltene Kolbenstange 4 und das Dosiseinstellglied 9. Das Reservoirteil 1 und der Mechanikhalter 3 bilden zusammen einen vorderen Gehäuseabschnitt des Injektionsgeräts. Mit diesem vorderen Gehäuseabschnitt ist ein hinterer Gehäuseabschnitt 11 verdreh- und verschiebegesichert verbunden. Der hintere Gehäuseabschnitt 11 bildet den Träger eines Dosier- und Betätigungselements 12 und zusammen mit demselben sowie einer Anzeigeeinrichtung 17 und weiteren Teilen des Injektionsgeräts das Dosier- und Betätigungsmodul 20.

Eine Dosier- und Betätigungsvorrichtung des Injektionsgeräts umfasst mit Ausnahme des Dosiseinstellglieds 9 und der Kolbenstange 4 die anderen Komponenten für die Auswahl der Produktdosis und die Betätigung des Injektionsgeräts. Insbesondere umfasst sie das Dosier- und Betätigungselement 12 und die Zähl- und Anzeigeeinrichtung 17 zum Zählen und optischen Anzeigen der ausgewählten Produktdosis. Nicht zuletzt der Zähl- und Anzeigeeinrichtung 17 wegen ist das Dosier- und Betätigungsmodul 20 ein hochwertiges und daher hochpreisiges Teil des Injektionsgeräts. Während das vergleichsweise preiswerte Reservoirmodul 10 als Einwegmodul konzipiert ist, das nach der Entleerung des Reservoirs einer Entsorgung zugeführt oder bei einem Hersteller wieder aufgearbeitet wird, ist das Dosier- und Betätigungsmodul 20 für die mehrmalige Verwendung mit immer wieder neuen Reservoirmodulen 10 bestimmt.

Für die Auswahl der Produktdosis wird das Dosier- und Betätigungselement 12 um die Längsachse L drehbar und ferner in und gegen die Vorschubrichtung V entlang der Längsachse L geradverschiebbar von dem hinteren Gehäuseabschnitt 11 gelagert. Das Dosier- und Betätigungselement 12 ist hohlzylindrisch und umgibt mit einem vorderen Abschnitt die Kolbenstange 4. Ferner ragt es mit dem vorderen Abschnitt in das hülsenförmige Dosiseinstellglied 9 ein. Ein hinterer Abschnitt des Dosier- und Betätigungselements 12 ragt über ein hinteres Ende des Gehäuseabschnitts 11 hinaus und wird von einer Kappe 14 verschlossen.
Eine Rückstellfeder 16 spannt das Dosier- und Betätigungselement 12 elastisch gegen die Vorschubrichtung V in die in Figur 2 dargestellte hintere Axialposition, die im Folgenden als Ausgangsposition bezeichnet wird. In der Ausgangsposition kann durch Drehung des Dosier- und Betätigungselements 12 um die Längsachse L die Dosisauswahl vorgenommen werden. Anschließend kann ebenfalls aus der Ausgangsposition durch Axialverschiebung des Dosier- und Betätigungselements 12 in die Vorschubrichtung V die Ausschüttung der ausgewählten Produktdosis bewirkt werden.

Das Dosiseinstellglied 9 und das Dosier- und Betätigungselement 12 sind aneinander axial geradgeführt und um die Längsachse L nicht verdrehbar miteinander verbunden. Im Falle einer Drehbewegung des Dosier- und Betätigungselements 12 wird das Dosiseinstellglied wegen der verdrehgesicherten Verbindung mit dem Dosier- und Betätigungselement 12 einerseits und dem Gewindeeingriff mit der relativ zu dem Mechanikhalter 3 nicht verdrehbaren und von der Blockiereinrichtung 8 gehaltenen Kolbenstange 4 andererseits in eine Bewegung versetzt, die sich aus einer rotatorischen Bewegungskomponente um die Längsachse L und einer translatorischen Bewegungskomponente entlang der Längsachse L zusammensetzt. Diese Bewegung wird im Folgenden als Dosierdrehbewegung des Dosiseinstellglieds 9 bezeichnet. Die Dosierdrehbewegung wird durch eine Verdrehsicherungseinrichtung so begrenzt, dass das Dosiseinstellglied 9 in eine erste Drehrichtung bis in eine vordere, erste Endposition und in eine entgegengerichtete, zweite Drehrichtung bis in eine hintere, zweite Endposition bewegt werden kann. Die Verdrehsicherungseinrichtung verhindert, dass das Dosiseinstellglied 9 über die beiden Endpositionen hinaus relativ zu dem Mechanikhalter 3 bewegt werden kann. Wenn das Dosiseinstellglied 9 seine erste Endposition einnimmt, ist die auswählbare Minimaldosis, im Ausführungsbeispiel die Nulldosis, eingestellt. Wenn das Dosiseinstellglied 9 die zweite Endposition einnimmt, ist die auswählbare Maximaldosis eingestellt. Die erste Endposition wird im Folgenden als "Stop Minimaldosis" und die zweite Endposition als "Stop Maximaldosis" bezeichnet.

Das Dosiseinstellglied 9 bildet mit einer rückwärtigen Stirnfläche einen Translationsanschlag 9c für das Dosier- und Betätigungselement 12. Eine Translationsbewegung des Dosier- und Betätigungselements 12 ist relativ zu dem Dosiseinstellglied 9 in die Vorschubrichtung V nur solange möglich, bis das Dosier- und Betätigungselement 12 gegen den Anschlag 9c zu liegen kommt. Sobald an dem Anschlag 9c Kontakt besteht, nimmt das Dosier- und Betätigungselement 12 bei einer weiterführenden Bewegung in die Vorschubrichtung V das Dosiseinstellglied 9 bis in eine vordere Endposition mit, die von einem Translationsanschlag 3c des Mechanikhalters 3 bestimmt wird. Das Dosiseinstellglied 9 wiederum nimmt wegen des Gewindeeingriffs die Kolbenstange 4 mit.

Aus dem in Figur 2 dargestellten Ausgangszustand des Injektionsgerät werden die Dosisauswahl und die Produktausschüttung vorgenommen. Das Dosier- und Betätigungselement 12 nimmt seine Ausgangsposition, d.h. seine hinterste Axialposition, und das Dosiseinstellglied 9 nimmt die Position "Stop Minimaldosis" ein. In der Position "Stop Minimaldosis" wird das Dosiseinstellglied 9 zum einen von der Verdrehsicherungseinrichtung und zum anderen von dem Translationsanschlag 3c, den der Mechanikhalter 3 bildet, an einer Bewegung in die Vorschubrichtung V gehindert. Der Anschlag 3c dient der Blockierung der reinen Axialbewegung, die das Dosiseinstellglied 9 und die Kolbenstange 4 bei einem Ausschütthub gemeinsam ausführen, während die Verdrehsicherungseinrichtung eine Drehbewegung des Dosiseinstellglieds 9 relativ zu der Kolbenstange 4 in eine über die Position "Stop Minimaldosis" hinausführende erste Drehrichtung verhindert.

Um die zu verabreichende Dosis auszuwählen, wird das Dosier- und Betätigungselement 12 um die Längsachse L verdreht. Wegen der verdrehgesicherten Verbindung nimmt das Dosier- und Betätigungselement 12 bei seiner Drehbewegung das Dosiseinstellglied 9 mit. Diese Dosierdrehbewegung des Dosiseinstellglieds 9 in die der ersten Drehrichtung entgegengesetzte zweite Drehrichtung führt wegen des Gewindeeingriffs mit der Kolbenstange 4 zu einer translatorischen Bewegung des Dosiseinstellglieds 9 entlang der Längsachse L entgegen der Vorschubrichtung V. Die translatorische Bewegung führt das Dosiseinstellglied 9 nicht nur relativ zu dem Mechanikhalter 3, sondern auch relativ zu dem Dosier- und Betätigungselement 12 aus. Hierdurch wird ein lichter Abstand zwischen dem von dem Dosiseinstellglied 9 gebildeten Anschlag 9c und einem von dem Dosier- und Betätigungselement 12 gebildeten Gegenanschlag, im Ausführungsbeispiel das freie vordere Ende des Dosier- und Betätigungselements 12, verringert. Im Verlauf dieser Dosierdrehbewegung des Dosiseinstellglieds 9 entspricht die jeweils eingenommene Axial- und Winkelposition des Dosiseinstellglieds 9 der Produktdosis, die bei einer Betätigung des Dosier- und Betätigungselements 12 ausgeschüttet würde. Die Zähl- und Anzeigeeinrichtung 17 zeigt diese Produktdosis an.

Nach der Auswahl der Produktdosis kann die ausgewählte Produktdosis durch Betätigung des Dosier- und Betätigungselements 12 ausgeschüttet werden. Die Betätigung erfolgt, indem das Dosier- und Betätigungselement 12 in die Vorschubrichtung V gedrückt wird. Dabei legt das Dosier- und Betätigungselement 12 einen ersten Teil seiner Wegstrecke alleine zurück, bis es gegen den Translationsanschlag 9c des Dosiseinstellglieds 9 zu liegen kommt. Im Zuge seiner weiteren Axialbewegung nimmt es dann das Dosiseinstellglied 9 und die Kolbenstange 4 mit, bis das Dosiseinstellglied 9 gegen den von dem Mechanikhalter 3 gebildeten Translationsanschlag 3c stößt. In diesem Moment ist der Ausschütthub beendet. Wird das Dosier- und Betätigungselement 12 losgelassen, so schiebt es aufgrund der gegen die Vorschubrichtung V andrückenden Elastizitätskraft der Rückstellfeder 16 zurück in seine in Figur 2 gezeigte Ausgangsposition, während die Kolbenstange 4 und das Dosiseinstellglied 9 wegen der Sperrwirkung der Blockiereinrichtung 8 die gerade eingenommene, neue Axialposition beibehalten. Durch die Ausführung des Ausschütthubs oder durch die Rücksetzbewegung der Kolbenstange 4 wird die Zähl- und Anzeigeeinrichtung 17 wieder auf die Minimaldosis zurückgesetzt, im Ausführungsbeispiel genullt. In der wieder erreichten Ausgangsposition des Dosier- und Betätigungselements 12 kann die Produktdosis für die nächste Ausschüttung ausgewählt werden. Der mechanische Eingriff zwischen dem Dosiseinstellglied 9 und der Kolbenstange 4 einerseits und dem Dosiseinstellglied 9 und dem Dosier- und Betätigungselement 12 andererseits wird im zusammengebauten Zustand des Injektionsgeräts in keiner Phase der Bewegungsabläufe gelöst. Die Figuren 3 bis 8 zeigen den Mechanikhalter 3 und das Dosiseinstellglied 9 und insbesondere die von diesen beiden Teilen gebildete Verdrehsicherungseinrichtung. Der Mechanikhalter 3 und das Dosiseinstellglied 9 sind je einstückige Hülsenkörper, wobei der Mechanikhalter 3, wie in Figur 2 erkennbar, das Dosiseinstellglied 9 in Bezug auf die Längsachse L konzentrisch umgibt.

Die Verdrehsicherungseinrichtung wird von Anschlagpaaren aus Verdrehanschlägen und Verdrehgegenanschlägen für die Position "Stop Minimaldosis" und von weiteren Anschlagpaaren aus Verdrehanschlägen und Vdrdrehgegenanschlägen für die Position "Stop Maximaldosis" gebildet. Die Blockierung in Bezug auf die Position "Stop Minimaldosis" wird von Verdrehanschlägen 9a des Dosiseinstellglied 9 und Verdrehgegenanschlägen 3a des Mechanikhalters 3 gebildet. Verdrehanschläge 3b des Mechanikhalters 3 und Verdrehgegenanschläge 9b des Dosiseinstellglieds 9 bilden die Blockierung für die Position "Stop Maximaldosis". Gekennzeichnet sind je mit einem Richtungspfeil auch die beiden Drehrichtungen D1 und D2 des Dosiseinstellglieds 9.

Die Verdrehanschläge 9a sind Anschlagflächen, die in die erste Drehrichtung D1 wirksam sind, indem sie in die erste Drehrichtung D1 weisen. Je ein Verdrehanschlag 9a wird von einem Schnapper 9s gebildet. Mehrere Schnapper 9s sind an einem hinteren Ende des Dosiseinstellglieds 9 an dessen äußeren Umfang gleichmäßig verteilt geformt. Jeder der Schnapper 9s ist als biegeelastische Zunge geformt, die in die erste Drehrichtung D1 um die Längsachse L gekrümmt von einem Mantelsegment des Dosiseinstellglieds 9 frei abragt und je eine Anschlagfläche 9a an ihrem in Bezug auf die erste Drehrichtung D1 vorderen, freien Ende bildet. Die Schnapper 9s ragen mit ihren freien Enden in Radialrichtung ein wenig über den Rest des Dosiseinstellglieds 9 hinaus. Jeder der Schnapper 9s ist auf die Längsachse L zu elastisch biegbar, kann also als Biegebalken angesehen werden, der sich von seinem Fußende, dem Ort fester Einspannung, bis zu seinem freien, die Anschlagfläche 9a bildenden Ende erstreckt.

Der Mechanikhalter 3 bildet die mit den Verdrehanschlägen 9a zusammenwirkenden Verdrehgegenschläge 3a durch Seitenwände, die den Verdrehanschlägen 3a zugewandt gegenüberliegen, wenn das Dosiseinstellglied 9 in die erste Drehrichtung D1 gedreht wird. Die Verdrehgegenanschläge 3a könnten alternativ durch Vorsprünge gebildet werden, die in solch einer alternativen Ausführung über eine ansonsten kreiszylindrische Innenmantelfläche des Mechanikhalters 3 nach innen in Richtung auf die Längsachse L vorragen würden. Insgesamt bildet der Mechanikhalter 3 vier Verdrehgegenanschläge 3a. In dem Hülsenmantel des Mechanikhalters 3, der im montierten Zustand das Dosiseinstellglied 9 umgibt, ist an in Bezug auf die Längsachse L diametral einander gegenüberliegenden Seiten je ein Fenster ausgenommen. In jedem der beiden Fensterbereiche sind zwei der vier Verdrehgegenanschläge 3a angeordnet. Den einen der Verdrehgegenanschläge 3a bildet eine Seitenwandung des betreffenden Fensters, den jeweils anderen bildet eine in das Fenster in axialer Richtung hineinragende Rippe 3r. Die derart gebildeten Verdrehgegenanschläge 3a sind die an der Seitenwandung und der Rippe 3r gegen die erste Drehrichtung D1 den Verdrehanschlägen 9a gegenüberliegend zugewandten, ebenen Gegenanschlagflächen, die in Bezug auf die Längsachse L je in einer Axial/Radial-Ebene liegen. Die Verdrehgegenanschläge 3a sind starr, d.h. nicht nachgiebig.

Für den Teil der Verdrehsicherungseinrichtung, der das Dosiseinstellglied 9 in der Position "Stop Maximaldosis" blockiert, bildet der Mechanikhalter 3 die Verdrehanschläge 3b an zwei elastischen Schnappern 3s. Je einer der Schnapper 3s ist in einem der in dem Mechanikhalter 3 ausgenommenen Fenster angeordnet. Die Schnapper 3s werden ebenfalls je von einer biegeelastischen Zunge gebildet, die von einer Seitenwandung ihres Fensters frei vorragen. Als Verdrehgegenanschlag 3b wird bei jedem der Schnapper 3s wie bereits bei den Schnappern 9s die am jeweils freien Schnapperende gebildete Anschlagfläche verstanden. Da die Schnapper 3s an dem Mechanikhalter 3 geformt sind, weisen ihre Anschlagflächen 3b ebenfalls in die erste Drehrichtung D1. Die beiden Anschlagflächen 3b bilden je eine Ebene, die schräg zu einer auf die Längsachse L bezogenen Axial/Radial-Ebene weist. An den Schnappern 3s ist je ein nach radial außen vorstehender Nocken geformt, der dazu dient, seinen Schnapper 3s im montierten Zustand des Injektionsgeräts (Figur 2) ein kleines Stück weit elastisch nach innen zu drücken. Ohne diesen Druck, d.h. vor dem Zusammenbau von Reservoirmodul 10 und Dosier- und Betätigungsmodul 12, sind die Verdrehanschläge 3b von den Verdrehgegenanschlägen 9b frei.

Die mit den Verdrehanschlägen 3b zusammenwirkenden Verdrehgegenanschläge 9b sind nicht nachgiebig an dem Dosiseinstellglied 9 geformt. Je ein Verdrehgegenanschlag 9b wird von einem Nocken 9n gebildet. Die Nocken 9n sind gleichmäßig über den Umfang verteilt an einer Mantelaußenfläche an einem vorderen Ende des Dosiseinstellglieds 9 nach radial auswärts vorragend gebildet. Die Nocken 9n bilden die Verdrehgegenanschläge 9b je als eine in die zweite Drehrichtung D2 weisende Gegenanschlagfläche 9b. Die Gegenanschlagflächen 9b weisen je unter dem gleichen Neigungswinkel wie die Anschlagflächen 3b schräg zu einer auf die Längsachse L bezogenen Axial/Radial-Ebene. Die Verdrehgegenanschläge bzw. Anschlagflächen 3b und die Verdrehanschläge bzw. Gegenanschlagflächen 9b wirken in der Art von schiefen Ebenen zusammen, wenn das Dosiseinstellglied 9 in die zweite Drehrichtung D2 in die Position "Stop Maximaldosis" bewegt wird.

Die Verdrehsicherungseinrichtung kann das Dosiseinstellglied 9 in mehreren Drehwinkelpositionen blockieren, wobei die Anzahl dieser Drehwinkelpositionen der Anzahl der über einen Drehwinkel von 360° diskret einstellbaren Dosen entspricht. Dies stellt sicher, dass das Dosiseinstellglied 9 über die blockierten Endpositionen hinaus um je höchstens eine einstellbare Dosiseinheit weitergedreht werden kann. Die Anschlagpaare 9a und 3a für die Position "Stop Minimaldosis" und die Anschlagpaare 3b und 9b für die Position "Stop Maximaldosis" sind in Abstimmung auf die Rastpositionen angeordnet, die das Dosiseinstellglied 9 bei der Dosisauswahl relativ zu dem Mechanikhalter 3 einnehmen kann. Die Abstimmung ist derart, dass das Dosiseinstellglied 9 in jeder seiner Rastpositionen ungehindert von der Verdrehsicherungseinrichtung die Ausschüttbewegung bis gegen den Translationsanschlag 3c des Mechanikhalters 3 ausführen kann.

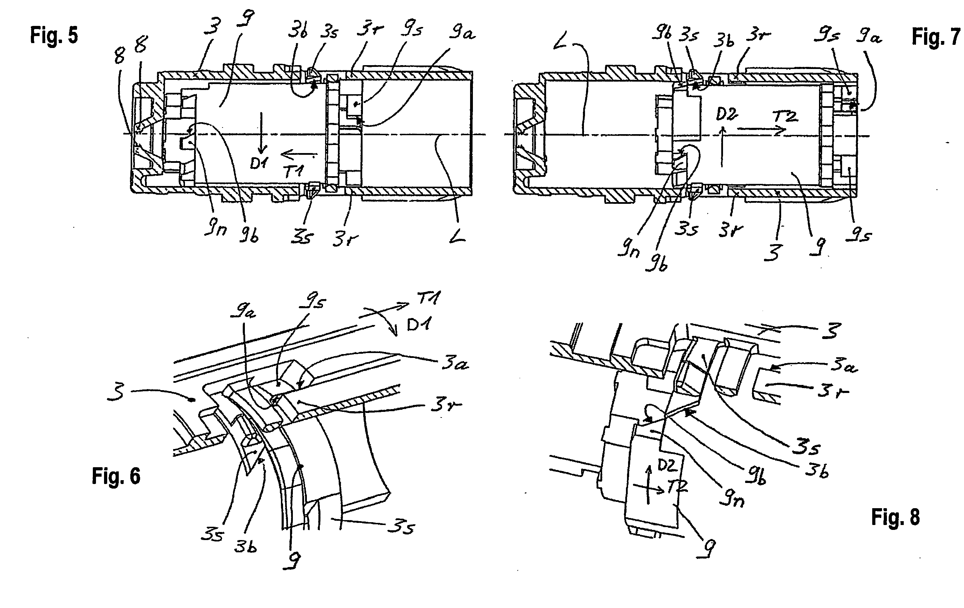

Die Figuren 5 und 6 zeigen den Mechanikhalter 3 und das Dosiseinstellglied 9 in der Position "Stop Minimaldosis" des Dosiseinstellglieds 9. Mit Richtungspfeilen angedeutet ist wieder die in diese Position führende erste Drehrichtung D1 und ferner die Richtung T1 der sich daraus ergebenden Translationsbewegung des Dosiseinstellglieds 9. Die Figur 6 zeigt für diese Position ein in Anschlag befindliches Paar aus einem der Verdrehanschläge 9a und einem der Verdrehgegenanschläge 3a.

Die Figuren 7 und 8 zeigen den Mechanikhalter 3 und das Dosiseinstellglied 9 in der Extremposition "Stop Maximaldosis" des Dosiseinstellglieds 9. Die zweite Drehrichtung D2, die in diese Endposition führt, ist wieder als Richtungspfeil D2 angedeutet. Die Richtung T2 der überlagerten Translationsbewegung ist ebenfalls eingezeichnet. Insbesondere in Figur 8 ist erkennbar, wie aufgrund der Neigung der Anschlagflächen 3b und 9b die Anschlagpaare bei Erreichen der Position "Stop Maximaldosis" einander "fangen", um anschließend in der Art von schiefen Ebenen aneinander abzugleiten. Diese kurze Gleitbewegung wird entweder durch Selbsthemmung oder von einem axial erstreckten, in Bezug auf die Richtung T2 der Translationsbewegung rückwärtigen Ende der Gegenanschlagfläche 9b beendet, d.h. die Gegenanschlagfläche 9b knickt zur Längsachse L ab.

Die Schnapper 9s und die Schnapper 3s geben bei der Drehbewegung des Dosiseinstellglieds 9, die aus der von ihnen jeweils blockierten Endposition herausführt, nach radial einwärts elastisch nach, wenn sie bei der Drehbewegung über ihre Verdrehgegenanschläge 3a und 9b streifen, so dass die Dosierdrehbewegung zwischen den beiden Endpositionen "Stop Minimaldosis" und "Stop Maximaldosis" nicht behindert wird.

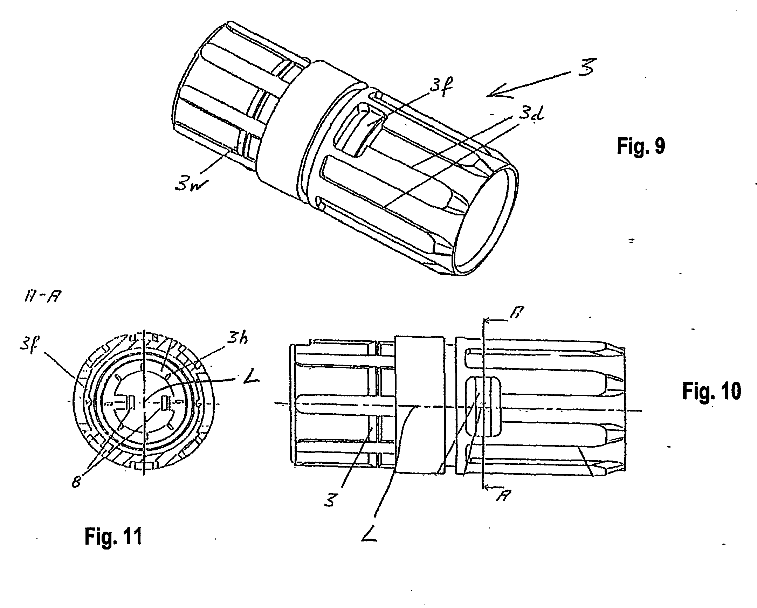

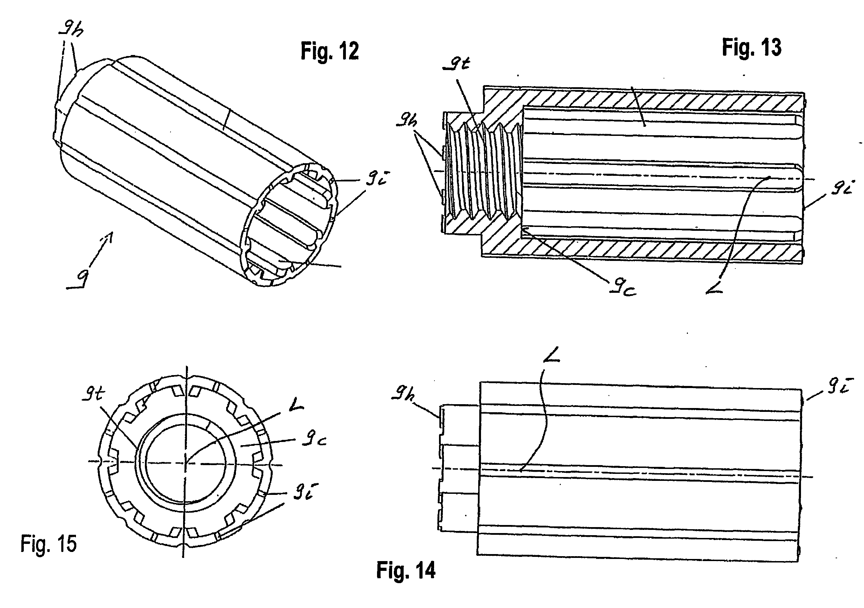

Die Figuren 9 bis 15 zeigen einen Mechanikhalter 3 und ein Dosiseinstellglied 9, die eine Verdrehsicherungseinrichtung für die Blockierung der beiden Endpositionen "Stop Minimaldosis" und "Stop Maximaldosis" nach einem zweiten Ausführungsbeispiel bilden. Die Verdrehanschläge und die Verdrehgegenanschläge werden in dem zweiten Ausführungsbeispiel für beide Endpositionen von axial aufeinander zu tragenden, nicht nachgiebigen Vorsprüngen gebildet. Für die Blockierung in der Position "Stop Minimaldosis" wirken die Verdrehanschläge 9h des Dosiseinstellglieds 9 mit den Verdrehgegenanschlägen 3h des Mechanikhalters 3 zusammen. Für die Blockierung in der Position "Stop Maximaldosis" wirken die Verdrehanschläge 9i des Dosiseinstellglieds 9 mit nicht eingezeichneten Verdrehgegenanschlägen des hinteren Gehäuseabschnitts 11 zusammen. Die Verdrehanschläge 9h ragen von einer vorderen Stirnfläche des Dosiseinstellglieds 9 in die Vorschubrichtung V ab. Die Verdrehanschläge 9i ragen entgegen der Vorschubrichtung V von einer hinteren Stirnfläche des Dosiseinstellglieds 9 ab. Entsprechend ragen die Verdrehgegenanschläge 3h den Verdrehanschlägen 9h entgegen von der Stirnfläche des Mechanikhalters 3 ab, die den Translationsanschlag 3c für den Ausschütthub bildet. Die Verdrehgegenanschläge für die Verdrehanschläge 9i ragen von einer radial einwärts ragenden Schulter des hinteren Gehäuseabschnitts 11 in die Vorschubrichtung V den Verdrehanschlägen 9i entgegen ab. Weitere Details der Verdrehsicherungseinrichtung des zweiten Ausführungsbeispiels und der weiteren Ausbildung des Mechanikhalters 3 und des Dosiseinstellglieds 9 des zweiten Ausführungsbeispiels werden in der DE 101 63 328 A1 beschrieben.

## Patentansprüche

1. Injektionsgerät mit endpositionsblockiertem Dosiseinstellglied, das Injektionsgerät umfassend:
a) ein Gehäuse (1, 3, 11) mit einem Reservoir für ein injizierbares Produkt,
b) eine Fördereinrichtung (2, 4, 12), mit der eine ausgewählte Produktdosis aus dem Reservoir ausschüttbar ist,
c) ein Dosiseinstellglied (9), das für die Auswahl der Produktdosis relativ zu dem Gehäuse (1, 3, 11) eine rotatorische Bewegung in eine erste Drehrichtung (D1) bis in eine erste Endposition und in eine entgegengesetzte zweite Drehrichtung (D2) bis in eine zweite Endposition ausführen kann und das mit der Fördereinrichtung (2, 4, 12) so gekoppelt ist, dass die Fördereinrichtung (2, 4, 12) bei einer Betätigung die mittels des Dosiseinstellglieds (9) ausgewählte Produktdosis ausschüttet,
d) und eine Verdrehsicherungseinrichtung (3a, 9a; 3b, 9b), die wenigstens einen ersten Verdrehanschlag (9a; 3b) und wenigstens einen ersten Verdrehgegenanschlag (3a; 9b) umfasst, von denen der eine von dem Gehäuse (1, 3, 11) und der andere von dem Dosiseinstellglied (9) gebildet wird,
e) wobei der erste Verdrehanschlag (9a; 3b) und der erste Verdrehgegenanschlag (3a; 9b) in der ersten Endposition gegeneinander in die erste Drehrichtung (D1) auf Anschlag sind, um die rotatorische Bewegung des Dosiseinstellglieds (9) in die erste Drehrichtung (D1) über die erste Endposition hinaus zu verhindern,
**dadurch gekennzeichnet, dass**
f) die Verdrehsicherungseinrichtung (3a, 9a; 3b, 9b) wenigstens einen zweiten Verdrehanschlag (3b) und wenigstens einen zweiten Verdrehgegenanschlag (9b) umfasst, von denen der eine von dem Gehäuse (1, 3, 11) und der andere von dem Dosiseinstellglied (9) gebildet wird,
g) und dass der zweite Verdrehanschlag (3b) und der zweite Verdrehgegenanschlag (9b) in der zweiten Endposition gegeneinander in die zweite Drehrichtung (D2) auf Anschlag sind, um die rotatorische Bewegung des Dosiseinstellglieds (9) in die zweite Drehrichtung (D2) über die zweite Endposition hinaus zu verhindern.

2. Injektionsgerät nach Anspruch 1, **dadurch gekennzeichnet, dass** das Dosiseinstellglied (9) entlang einer Drehachse (L) seiner rotatorischen Bewegung zwischen der Endposition und einer weiteren Endposition relativ zu dem Gehäuse (1, 3, 11) hin und her bewegbar ist.

3. Injektionsgerät nach einem der vorhergehenden Ansprüche, **dadurch gekennzeichnet, dass** das Dosiseinstellglied (9) in einem Stück oder aus in Bezug auf die rotatorische Bewegung miteinander permanent verdrehgesichert verbundenen Stücken gebildet ist.

4. Injektionsgerät nach einem der vorhergehenden Ansprüche, **dadurch gekennzeichnet, dass** die Fördereinrichtung (2, 4, 12) eine Kolbenstange (4) umfasst, die für die Ausführung eines Ausschütthubs entlang einer Drehachse (L) der rotatorischen Bewegung des Dosiseinstellglieds (9) in eine Vorschubrichtung (V) bewegbar ist und ein Dosiergewinde (5) um die Drehachse (L) aufweist, und dass das Dosiseinstellglied (9) ein Dosiergegengewinde (9t) aufweist, das für die Auswahl der Produktdosis mit dem Dosiergewinde (5) der Kolbenstange (4) in einem Gewindeeingriff ist.

5. Injektionsgerät nach einem der vorhergehenden Ansprüche, **dadurch gekennzeichnet, dass** ein Schnapper (9s; 3s), der in eine Richtung elastisch nachgiebig ist, die in Bezug auf eine Drehachse (L) der rotatorischen Bewegung des Dosiseinstellglieds (9) radial weist oder zumindest eine radiale Richtungskomponente hat, zumindest einen der Verdrehanschläge (9a; 3b) bildet.

6. Injektionsgerät nach dem vorhergehenden Anspruch, **dadurch gekennzeichnet, dass** der Schnapper (9s; 3s) eine radial in Bezug auf die Drehachse (L) biegeelastische Zunge ist, die von dem Gehäuse (1, 3, 11) oder dem Dosiseinstellglied (9) abragt.

7. Injektionsgerät nach einem der zwei vorhergehenden Ansprüche, **dadurch gekennzeichnet, dass** der Schnapper (9s; 3s) sich zumindest im Wesentlichen tangential in Bezug auf die Drehachse (L), d.h. zumindest im Wesentlichen je senkrecht zu der Drehachse (L) und einer Radialen auf die Drehachse (L), erstreckt.

8. Injektionsgerät nach dem vorhergehenden Anspruch, **dadurch gekennzeichnet, dass** der Schnapper (9s; 3s) sich in Bezug auf die Drehachse (L) in Umfangsrichtung erstreckt.

9. Injektionsgerät nach einem der vorhergehenden Ansprüche, **dadurch gekennzeichnet, dass** ein hülsenförmiger Abschnitt (3) des Gehäuses (1, 3, 11) und ein zylindrischer Abschnitt des Dosiseinstellglieds (9) einander um die Drehachse (L) umgeben.

10. Injektionsgerät nach dem vorhergehenden Anspruch, **dadurch gekennzeichnet, dass** an einem der Abschnitte (3, 9) ein den ersten oder den zweiten Verdrehanschlag (9a; 3b) bildender, elastischer Schnapper (9s; 3s) geformt und in eine Richtung, die in Bezug auf eine Drehachse (L) der rotatorischen Bewegung des Dosiseinstellglieds (9) radial weist oder eine radiale Richtungskomponente hat, gegen den anderen der Abschnitte (3, 9) elastisch gespannt ist.

11. Injektionsgerät nach dem vorhergehenden Anspruch, **dadurch gekennzeichnet, dass** ein Vorsprung, der von dem anderen der Abschnitte (3, 9) in Richtung auf den einen der Abschnitte (3, 9) vorragt, den Verdrehgegenanschlag (3a; 9b) für den Schnapper (9s; 3s) bildet.

12. Injektionsgerät nach einem der vorhergehenden Ansprü0che, **dadurch gekennzeichnet, dass** wenigstens einer der Verdrehgegenanschläge (3a; 9b) starr ist.

13. Injektionsgerät nach einem der vorhergehenden Ansprüche, **dadurch gekennzeichnet, dass** der erste Verdrehanschlag (9a) mit dem Dosiseinstellglied (9) und der erste Verdrehgegenanschlag (3a) mit dem Gehäuse (1, 3, 11) in einem Stück geformt sind.

14. Injektionsgerät nach einem der vorhergehenden Ansprüche, **dadurch gekennzeichnet, dass** der zweite Verdrehanschlag (3b) mit dem Gehäuse (1, 3, 11) und der zweite Verdrehgegenanschlang (9b) mit dem Dosiseinstellglied (9) in einem Stück geformt sind.

15. Injektionsgerät nach einem der vorhergehenden Ansprüche, **dadurch gekennzeichnet, dass** der erste Verdrehanschlag (9a) und der erste Verdrehgegenanschlag (3a) von Anschlagflächen gebildet werden, die sich zumindest im Wesentlichen parallel zu einer Drehachse (L) der rotatorischen Bewegung des Dosiseinstellglieds (9) erstrecken.

16. Injektionsgerät nach einem der vorhergehenden Ansprüche, **dadurch gekennzeichnet, dass** der zweite Verdrehanschlag (3b) und der zweite Verdrehgegenanschlag (9b) von Anschlagflächen gebildet werden, die in der Art von schiefen Ebenen bis in die Endposition aneinander abgleiten.

## Claims

1. An injection apparatus with an end position-blocked dosage setting member, said injection apparatus comprising:
a) a casing (1, 3, 11) comprising a reservoir for an injectable product;
b) a conveying means (2, 4, 12) using which a selected product dosage can be delivered from the reservoir;
c) a dosage setting member (9) which in order to select the product dosage can perform a rotational movement in a first rotational direction (D1) up to a first end position and in an opposite, second rotational direction (D2) up to a second end position, relative to the casing (1, 3, 11), and which is coupled to the conveying means (2,4,12) such that when activated, the conveying means (2,4,12) delivers the product dosage selected by means of the dosage setting member (9);
d) and a rotational block (3a, 9a; 3b, 9b) which comprises at least a first rotational stopper (9a; 3b) and at least a first rotational counter stopper (3a; 9b), one of which is formed by the casing (1, 3, 11) and the other of which is formed by the dosage setting member (9);
e) wherein the first rotational stopper (9a; 3b) and the first rotational counter stopper (3a; 9b) abut against each other in the first end position in the first rotational direction (D1), in order to prevent the dosage setting member (9) from rotating in the first rotational direction (D1) beyond the first end position;
**characterised in that**:
f) the rotational block (3a, 9a; 3b, 9b) comprises at least a second rotational stopper (3b) and at least a second rotational counter stopper (9b), one of which is formed by the casing (1, 3, 11) and the other of which is formed by the dosage setting member (9);
g) and **in that** the second rotational stopper (3b) and the second rotational counter stopper (9b) abut against each other in the second end position in the second rotational direction (D2), in order to prevent the dosage setting member (9) from rotating in the second rotational direction (D2) beyond the second end position.

2. The injection apparatus according to claim 1, **characterised in that** the dosage setting member (9) can be moved back and forth along a rotational axis (L) of its rotational movement between the end position and another end position, relative to the casing (1, 3,11).

3. The injection apparatus according to any one of the preceding claims, **characterised in that** the dosage setting member (9) is formed in one piece or is formed from pieces which are permanently connected to each other non-rotationally with respect to the rotational movement.

4. The injection apparatus according to any one of the preceding claims, **characterised in that** the conveying means (2, 4, 12) comprises a piston rod (4) which can be moved in an advancing direction (V) along a rotational axis (L) of the rotational movement of the dosage setting member (9) in order to perform a delivery stroke, and comprises a dosing thread (5) about the rotational axis (L), and **in that** the dosage setting member (9) comprises a dosing counter thread (9t) which is in threaded engagement with the dosing thread (5) of the piston rod (4) in order to select the product dosage.

5. The injection apparatus according to any one of the preceding claims, **characterised in that** a latch (9s; 3s), which is elastically flexible in a direction which points radially with respect to a rotational axis (L) of the rotational movement of the dosage setting member (9) or at least has a radial direction component, forms at least one of the rotational stoppers (9a; 3b).

6. The injection apparatus according to the preceding claim, **characterised in that** the latch (9s; 3s) is a tongue which elastically bends radially with respect to the rotational axis (L) and which projects from the casing (1, 3, 11) or the dosage setting member (9).

7. The injection apparatus according to any one of the preceding two claims, **characterised in that** the latch (9s; 3s) extends at least substantially tangentially with respect to the rotational axis (L), i.e. at least substantially perpendicular to each of the rotational axis (L) and a radial onto the rotational axis (L).

8. The injection apparatus according to the preceding claim, **characterised in that** the latch (9s; 3s) extends in the circumferential direction with respect to the rotational axis (L).

9. The injection apparatus according to any one of the preceding claims, **characterised in that** a sleeve-shaped section (3) of the casing (1, 3, 11) and a cylindrical section of the dosage setting member (9) surround each other about the rotational axis (L).

10. The injection apparatus according to the preceding claim, **characterised in that** an elastic latch (9s; 3s) forming the first or second rotational stopper (9a; 3b) is formed on one of the sections (3, 9) and is elastically tensed against the other section (3, 9) in a direction which points radially with respect to a rotational axis (L) of the rotational movement of the dosage setting member (9) or has a radial direction component.

11. The injection apparatus according to the preceding claim, **characterised in that** a protrusion, which protrudes from the second section (3, 9) towards the first section (3, 9), forms the rotational counter stopper (3a; 9b) for the latch (9s; 3s).

12. The injection apparatus according to any one of the preceding claims, **characterised in that** at least one of the rotational counter stoppers (3a; 9b) is rigid.

13. The injection apparatus according to any one of the preceding claims, **characterised in that** the first rotational stopper (9a) is formed in one piece with the dosage setting member (9) and the first rotational counter stopper (3a) is formed in one piece with the casing (1,3,11).

14. The injection apparatus according to any one of the preceding claims, **characterised in that** the second rotational stopper (3b) is formed in one piece with the casing (1, 3, 11) and the second rotational counter stopper (9b) is formed in one piece with the dosage setting member (9).

15. The injection apparatus according to any one of the preceding claims, **characterised in that** the first rotational stopper (9a) and the first rotational counter stopper (3a) are formed by stopper areas which extend at least substantially parallel to a rotational axis (L) of the rotational movement of the dosage setting member (9).

16. The injection apparatus according to any one of the preceding claims, **characterised in that** the second rotational stopper (3b) and the second rotational counter stopper (9b) are formed by stopper areas which slide off of each other in the manner of inclined planes, up to the end position.

## Revendications

1. Appareil d'injection comprenant un organe de réglage de dose bloqué en position finale, l'appareil d'injection comportant :
a) un boîtier (1, 3, 11) comprenant un réservoir pour un produit injectable,
b) un système d'amenée (2, 4, 12), avec lequel une dose de produit sélectionnée peut être déversée du réservoir,
c) un organe de réglage de dose (9), qui, pour sélectionner la dose de produit peut réaliser par rapport au boîtier (1, 3, 11) un déplacement rotatif dans une première direction de rotation (D1) jusque dans une première position finale et dans une seconde direction de rotation (D2) opposée jusque dans une seconde position finale et qui est couplé au système d'amenée (2, 4, 12), de sorte que le système d'amenée (2, 4, 12), lorsqu'il est actionné, déverse la dose de produit sélectionnée au moyen de l'organe de réglage de dose (9),
d) et un dispositif de sécurité de rotation (3a, 9a ; 3b, 9b) qui comporte au moins une première butée de rotation (9a ; 3b) et au moins une première contre-butée de rotation (3a ; 9b), parmi lesquels l'une est formée par le boîtier (1, 3, 11) et l'autre par l'organe de réglage de dose (9),
e) la première butée de rotation (9a ; 3b) et la première contre-butée de rotation (3a ; 9b) dans la première position finale étant en butée l'une contre l'autre dans la première direction de rotation (D1), afin d'empêcher le déplacement rotatif de l'organe de réglage de dose (9) dans la première direction de rotation (D1) au-delà de la première position finale,
**caractérisé en ce que**
f) le dispositif de sécurité de rotation (3a, 9a ; 3b, 9b) comporte au moins une seconde butée de rotation (3b) et au moins une seconde contre-butée de rotation (9b), parmi lesquelles l'une est formée par le boîtier (1, 3, 11) et l'autre par l'organe de réglage de dose (9),
g) et **en ce que** la seconde butée de rotation (3b) et la seconde contre-butée de rotation (9b) dans la seconde position finale sont en butée l'une contre l'autre dans la seconde direction de rotation (D2), afin d'empêcher le déplacement rotatif de l'organe de réglage de dose (9) dans la seconde direction de rotation (D1) au-delà de la seconde position finale.

2. Appareil d'injection selon la revendication 1, **caractérisé en ce que** l'organe de réglage de dose (9) peut être animé d'un mouvement de va-et-vient le long d'un axe de rotation (L) de son déplacement rotatif entre la position finale et une autre position finale par rapport au boîtier (1, 3, 11).

3. Appareil d'injection selon l'une des revendications précédentes, **caractérisé en ce que** l'organe de réglage de dose (9) est formé en une seule pièce ou de pièces reliées, de façon sécurisée en rotation, en permanence les unes aux autres avec une rotation limitée par rapport au déplacement rotatif.

4. Appareil d'injection selon l'une des revendications précédentes, **caractérisé en ce que** le système d'amenée (2, 4, 12) comporte une tige de piston (4), qui, pour réaliser une course de déversement, est mobile le long d'un axe de rotation (L) du déplacement rotatif de l'organe de réglage de dose (9) dans une direction d'avancée (V) et comprend un filet de dosage (5) autour de l'axe de rotation (L), et **en ce qu'**un organe de réglage de dose (9) comprend un contre-filet de dosage (9t), qui, pour sélectionner la dose de produit, est en prise filetée avec le filet de dosage (5) de la tige de piston (4).

5. Appareil d'injection selon l'une des revendications précédentes, **caractérisé en ce qu'**un loquet (9s ; 3s), qui est élastiquement flexible dans une direction, qui est orientée dans le sens radial par rapport à un axe de rotation (L) du déplacement rotatif de l'organe de réglage de dose (9) ou présente au moins une composante de direction radiale, forme au moins une des butées de rotation (9a ; 3b).

6. Appareil d'injection selon l'une des revendications précédentes, **caractérisé en ce que** le loquet (9s ; 3s) est une languette à flexion élastique dans le sens radial par rapport à l'axe de rotation (L), qui dépasse du boîtier (1, 3, 11) ou de l'organe de réglage de dose (9).

7. Appareil d'injection selon l'une des deux revendications précédentes, **caractérisé en ce que** le loquet (9s ; 3s) s'étend au moins sensiblement de manière tangentielle par rapport à l'axe de rotation (L), c'est-à-dire au moins sensiblement respectivement perpendiculairement à l'axe de rotation (L) et à une radiale sur l'axe de rotation (L).

8. Appareil d'injection selon l'une des revendications précédentes, **caractérisé en ce que** le loquet (9s ; 3s) s'étend dans la direction périphérique par rapport à l'axe de rotation (L).

9. Appareil d'injection selon l'une des revendications précédentes, **caractérisé en ce qu'**une section en forme de douille (3) du boîtier (1, 3, 11) et une section cylindrique de l'organe de réglage de dose (9) s'entourent mutuellement autour de l'axe de rotation (L).

10. Appareil d'injection selon l'une des revendications précédentes, **caractérisé en ce qu'**un loquet (9s ; 3s) élastique, formant une première ou seconde butée de rotation (9a ; 3b), est réalisé sur une des sections (3, 9) et est pressé de manière élastique contre l'autre des sections (3, 9) dans une direction qui est orientée dans le sens radial ou présente une composante de direction radiale par rapport à un axe de rotation (L) du déplacement rotatif de l'organe de réglage de dose (9).

11. Appareil d'injection selon l'une des revendications précédentes, **caractérisé en ce qu'**une saillie qui dépasse de l'autre des sections (3, 9) en direction d'une des sections (3, 9) forme la contre-butée de rotation (3a ; 9b) pour le loquet (9s ; 3s).

12. Appareil d'injection selon l'une des revendications précédentes, **caractérisé en ce qu'**au moins une des contre-butées de rotation (3a ; 9b) est rigide.

13. Appareil d'injection selon l'une des revendications précédentes, **caractérisé en ce que** la première butée de rotation (9a) est formée en une seule pièce avec l'organe de réglage de dose (9) et la première contre-butée de rotation (3a) en une seule pièce avec le boîtier (1, 3, 11).

14. Appareil d'injection selon l'une des revendications précédentes, **caractérisé en ce que** la seconde butée de rotation (3b) est formée en une seule pièce avec le boîtier (1, 3, 11) et la seconde contre-butée de rotation (9b) en une seule pièce avec l'organe de réglage de dose (9).

15. Appareil d'injection selon l'une des revendications précédentes, **caractérisé en ce que** la première butée de rotation (9a) et la première contre-butée de rotation (3a) sont formées par des surfaces de butée qui s'étendent au moins sensiblement parallèlement à un axe de rotation (L) du déplacement rotatif de l'organe de réglage de dose (9).

16. Appareil d'injection selon l'une des revendications précédentes, **caractérisé en ce que** la seconde butée de rotation (3b) et la seconde contre-butée de rotation (9b) sont formées par des surfaces de butée qui glissent l'une sur l'autre à la façon de plans inclinés jusque dans la position finale.
